(19) Europäisches Patentamt
European Patent Office
Office européen des brevets

(11) Veröffentlichungsnummer: **0 273 109**
**B1**

(12) # EUROPÄISCHE PATENTSCHRIFT

(45) Veröffentlichungstag der Patentschrift: **16.08.90**

(51) Int. Cl.[5]: **C07C 45/62,** C07C 49/637

(21) Anmeldenummer: **87113740.2**

(22) Anmeldetag: **19.09.87**

(54) **Verfahren zur Herstellung von 4.4.8.8.10-Pentamethylbicyclo[4.4.0]decen-(1.6)-on(2).**

(30) Priorität: **22.11.86 DE 3639921**

(43) Veröffentlichungstag der Anmeldung: **06.07.88 Patentblatt 88/27**

(45) Bekanntmachung des Hinweises auf die Patenterteilung: **16.08.90 Patentblatt 90/33**

(84) Benannte Vertragsstaaten: **DE FR GB IT**

(56) Entgegenhaltungen:
**EP-A- 0 012 436**
**CH-A- 595 311**
**US-A- 4 377 714**

**CHEMICAL ABSTRACTS, Band 58, Nr. 7, 1. April 1963, Zusammenfassungsnr. 6712h, Columbus, Ohio, US; J. SYLVESTRE et al.: "Bicyclic dienones from substituted 1,3-dichloro-2-butenes"**

(73) Patentinhaber: **HÜLS AKTIENGESELLSCHAFT, Patentabteilung / PB 15 - Postfach 13 20, D-4370 Marl 1(DE)**

(72) Erfinder: **Büschken, Wilfried, Dr., Rosenkamp 10, D-4358 Haltern 6(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 273 109 B1

ACTORUM AG

## Beschreibung

Die Erfindung betrifft ein Verfahren zur Herstellung von 4.4.8.8.10-Pentamethylbicyclo[4.4.0]-decen-(1,6)-on(2) [A] aus einem $C_{15}$-Keton-Gemisch, wie man es durch Fraktionierung des Sumpfproduktes, das bei der Herstellung von Isophoron, vorzugsweise bei der Flüssigphasenreaktion, anfällt, bei 1 bis 100 mbar in ca. 30 %iger Ausbeute erhält, durch selektive Hydrierung.

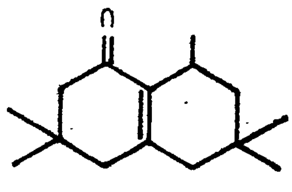

A

Die Verbindung A ist eine geeignete Vorstufe für die Synthese von Aminen, Diaminen, Heterocyclen und einer Vielzahl anderer Stoffe. Weiterhin kann sie als Riechstoff Verwendung finden. Bei der Herstellung von Isophoron durch Kondensation von Aceton entstehen bis zu 10 % höhermolekulare Kondensationsprodukte, die zur Zeit verbrannt werden. Es bestand daher die Aufgabe, dieses Gemisch in Wertprodukten aufzutrennen oder dazu umzusetzen.

Diese Aufgabe wird erfindungsgemäß entsprechend den Angaben der Patentansprüche gelöst.

Überraschenderweise wurde gefunden, daß man durch technisch leicht durchführbare Verfahrensschritte die obige Verbindung A in guter Ausbeute erhalten kann, indem man eine spezielle Fraktion des Isophoron-Sumpfproduktes selektiv hydriert.

Durch Fraktionierung des Isophoronsumpfproduktes bei 1 bis 100 mbar, vorzugsweise bei 10 bis 30 mbar erhält man einen Ketonschnitt, der im wesentlichen aus $C_{15}$-Verbindungen besteht. Bei einem Druck von 13 mbar siedet dieser Ketonschnitt, den man in einer Ausbeute von ca. 30 % erhält, bei 125 bis 145 °C. Als Hauptkomponenten enthält dieser Schnitt die Isomeren B und C

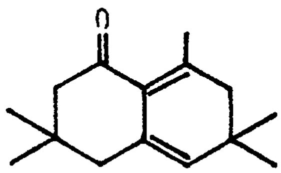

B

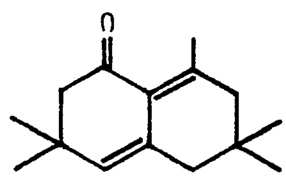

C

Die Lage der Doppelbindungen in B und C ist nicht vollständig abgesichert.

Das Verhältnis der beiden Isomeren B und C zueinander ist nicht konstant. Ebenfalls kann der Gehalt der beiden Stoffe in der $C_{15}$-Fraktion unterschiedlich sein. Die Summe bei den Isomeren liegt bei 75 bis 90 %, bezogen auf die $C_{15}$-Fraktion.

Die Hydrierung des $C_{15}$-Ketonschnitts liefert im allgemeinen ein sehr komplexes Reaktionsgemisch. Bei Verwendung von Nickelkontakten werden aus den Verbindungen B und C die entsprechend gesättigten Alkohole (bis zu 16 Isomeren) erhalten. Bei der Hydrierung der Verbindungen B und C an einem Palladiumkontakt bei höheren Temperaturen und Drücken entstehen die gesättigten Ketone (bis zu 8 Isomeren).

Überraschenderweise wurde gefunden, daß die Verbindungen B und C selektiv, fast in quantitativer Ausbeute zum gleichen Keton A hydriert werden können, wenn man bei tiefen Temperaturen von 25 bis 150 °C und Wasserstoffdrücken von 20 bis 250 bar an einem Edelmetallkontakt hydriert.

Bevorzugt hydriert man bei Temperaturen von 80 bis 100 °C. Vorzugsweise führt man die Hydrierung bei Wasserstoffdrücken von 80 bis 150 bar durch. Als Hydrierkontakte sind Edelmetallkontakte geeignet, wie z. B. Kontakte der Platingruppe, beispielsweise Palladium, Platin. Man kann handelsübliche Kontakte einsetzen, wie z. B. Pd/C oder $Pd/Al_2O_3$. Diese Kontakte enthalten im allgemeinen 0,1 bis 2 %, vorzugsweise 0,5 bis 2 Gew.-% Edelmetall. Bevorzugt setzt man Palladiumkontakte ein.

Durch Zusatz von Alkoholen, insbesondere Methanol, kann man geringe Überhydrierungen der Verbindung A vermeiden. Den Alkohol setzt man bevorzugt in Mengen von 10 bis 60 %, bezogen auf den Hydriereinsatz, zu.

Die Hydrierung führt man kontinuierlich oder diskontinuierlich durch. Ein Teil der Nebenprodukte wird zu Stoffen mit einer anderen Siedelage als das Keton A hydriert, so daß man durch Rektifikation den größten Teil der Begleitstoffe abtrennen kann.

Durch die selektive Hydrierung erhält man unter Hydrierbedingungen stabile Produkte. Die Hydrierzeit ist daher nicht kritisch. Durch eine Nachhydrierung wird das 4.4.8.8.10-Pentamethylbicyclo/4.4.0/decen-(1.6)-on(2) nicht weiter hydriert, wie die Beispiele 8 und 9 zeigen.

Beispiel 1

Man fraktioniert das Sumpfprodukt der Isophoronsynthese (durch Kondensation von Aceton) bei einem Druck von 13 mbar. Hierbei erhält man in 30 %iger Ausbeute einen Ketonschnitt ($Kp_{13}$ 125 bis 145 °C), der im wesentlichen aus $C_{15}$-Verbindungen besteht mit den Hauptkomponenten B und C.
Eine Lösung aus 300 g dieses $C_{15}$-Ketonschnitts (64 % B; 12,6 % C) und 700 ml Methanol wurden in einem 2-l-Autoklaven an 50 g Kontakt 2 % Pd/C 3 h bei einer Temperatur von 80 °C und bei einem Druck von 100 bar hydriert. Nach dem Abfiltrieren des Katalysators und Abziehen des Methanols wurde eine Lösung mit 76,6 % A, 0 % B und 0 % C erhalten. Durch Rektifikation erhält man die reine Verbindung A.

Beispiel 2

1000 g eines nach den Angaben des Beispiels 1 erhaltenen $C_{15}$-Ketonschnitts (65 % B; 13,3 % C) wurden in einem 2-l-Autoklaven an 50 g Kontakt 2 % Pd/C 3 h bei 80 °C und bei einem Druck von 100 bar hydriert. Nach dem Abfiltrieren des Katalysators wurde eine Lösung mit 77,8 % A, 0 % B und 0 % C erhalten.

Beispiel 3

100 ml eines $C_{15}$-Ketonschnitts (58,9 % B, 16,5 % C), erhalten nach den Angaben des Beispiels 1, wurden in einem 200-ml-Autoklaven an 5 g Kontakt 2 % Pd/C 5 h bei 80 °C und bei einem Druck von 145 bar hydriert. Nach dem Abtrennen des Katalysators wurde ein Filtrat mit 70 % A, 0 % B und 0 % C erhalten.

Beispiel 4

Eine Lösung aus 70 ml $C_{15}$-Ketonschnitt (58,9 % B; 16 % C) und 30 ml Methanol wurde in einem 200-ml-Autoklaven an 5 g Kontakt 2 % Pd/C 5 h bei 80 °C und einem Druck von 145 bar hydriert. Nach dem Abtrennen des Katalysators und Abziehen des Methanols wird eine Lösung mit 73,5 % A, 0 % B und 0 % C erhalten.

Beispiel 5

100 ml $C_{15}$-Ketonschnitt (58,9 % B; 16,5 % C) wurden in einem 200-ml-Autoklaven an 5 g Kontakt 0,5 % $Pd/Al_2O_3$ 5 h bei 80 °C und einem Druck von 145 bar hydriert. Nach dem Abfiltrieren des Katalysators wurde ein Filtrat mit 69,3 % A, 0 % B und 0 % C erhalten.

Beispiel 6

Eine Lösung aus 70 ml $C_{15}$-Ketonschnitt (58,9 % B; 16,5 % C) und 30 ml Methanol wurde in einem 200-ml-Autoklaven an 5 g Kontakt 0,5 % $Pd/Al_2O_3$ 5 h bei 80 °C und bei einem Druck von 145 bar hydriert. Nach dem Abfiltrieren des Katalysators und Abziehen des Methanols wurde eine Lösung mit 74,3 % A, 0 % B und 0 % C erhalten.

Beispiel 7 (Nachhydrierung)

100 ml 89,2 %iges A wurden in einem 200-ml-Autoklaven an 5 g Kontakt 0,5 % $Pd/Al_2O_3$ 5 h bei 80 °C und einem Druck von 145 bar hydriert. Nach der Abtrennung des Katalysators wurde eine Lösung mit 82,7 % an A erhalten.

Beispiel 8 (Nachhydrierung)

Eine Lösung aus 70 ml 89,2 %igem A und 30 ml Methanol wurde in einem 200-ml-Autoklaven an 5 g Kontakt 0,5 % $Pd/Al_2O_3$ 5 h bei 80 °C und bei einem Druck von 145 bar hydriert. Nach dem Abtrennen des Katalysators und Abziehen des Methanols wurde eine Lösung mit 89,1 % an A erhalten.

## Patentansprüche

1. Verfahren zur Herstellung von 4.4.8.8.10-Pentamethylbicyclo[4.4.0]-decen-(1.6)-on(2), dadurch gekennzeichnet, daß man das Sumpfprodukt, das bei der Herstellung von Isophoron anfällt, bei Drücken von 1 bis 100 mbar fraktioniert, den erhaltenen, im wesentlichen aus $C_{15}$-Verbindungen bestehenden, Ketonschnitt, der als Hauptkomponenten die Isomeren B und C enthält, isoliert und

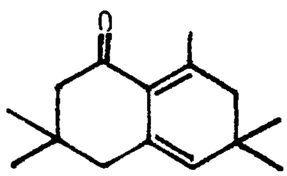

B

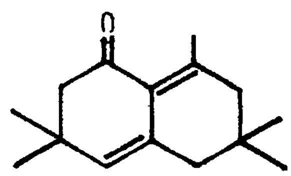

C

in Gegenwart eines Edelmetallkontaktes bei Wasserstoffdrücken von 20 bis 250 bar und Temperaturen von 25 bis 150 °C selektiv hydriert und den Hydrieraustrag rektifiziert.

2. Verfahren nach Anspruch 1, dadurch gekennzeichnet, daß man als Edelmetallkontakte Palladiumkontakte einsetzt.

3. Verfahren nach den Ansprüchen 1 und 2, dadurch gekennzeichnet, daß man bei Wasserstoffdrücken von 80 bis 150 bar hydriert.

4. Verfahren nach einem der Ansprüche 1 bis 3, dadurch gekennzeichnet, daß man bei Temperaturen von 80 bis 100 °C hydriert.

5. Verfahren nach einem der Ansprüche 1 bis 4, dadurch gekennzeichnet, daß man nach Zusatz eines Alkohols, vorzugsweise Methanol, hydriert.

## Claims

1. A process for the production of 4,4,8,8,10-pentamethylbicyclo-[4,4 0]-dec-(1,6)-en-(2)-one characterized by the fact that the bottom product which occurs during the production of isophorone is fractionated at a pressure of 1 to 100 mbar and the ketone fraction, consisting substantially of $C_{15}$-compounds and containing the isomers B and C as the

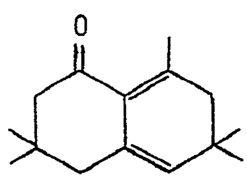

B

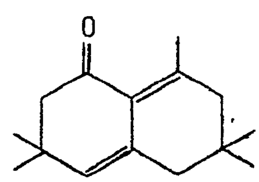

C

main components, is isolated and selectively hydrogenated in the presence of a noble metal contact catalyst at a hydrogen pressure between 20 and 250 bar and at a temperature between 25 and 150°C, and the hydrogenated product is rectified.

2. A process according to Claim 1, characterized in that a palladium contact catalyst is used as the noble metal contact catalyst.

3. A process according to Claims 1 and 2, characterized in that hydrogenation is performed at a hydrogen pressure of between 80 and 150 bar.

4. A process according to any one of Claims 1 to 3, characterized in that hydrogenation is performed at a temperature between 80 and 100°C.

5. A process according to an one of Claims 1 to 4, characterized in that hydrogenation is performed after addition of an alcohol, preferably methanol.

## Revendications

1. Procédé de fabrication de 4,4,8,8,10-pentaméthylbicyclo[4,4,0]-décène-(1,6)one(2) caractérisé en ce qu'on fractionne le produit de pied formé lors de la préparation d'isophorone, entre 1 et 100 mbars, on isole la coupe cétonique constituée pour l'essentiel de composés $C_{15}$, qui comprend comme composants principaux les isomères B et C:

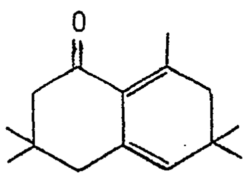

B

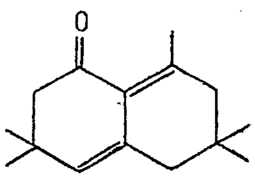

C

et on hydrogène sélectivement en présence d'un catalyseur à base de métaux précieux sous des pressions d'hydrogène de 20 à 250 mbars à des températures comprises entre 25 et 150°C et on rectifie le produit hydrogéné.

2. Procédé selon la revendication 1, caractérisé en ce que l'on utilise comme catalyseurs de métaux précieux des catalyseurs de palladium.

3. Procédé selon les revendications 1 et 2, caractérisé en ce qu'on hydrogène sous des pressions d'hydrogène comprises entre 80 et 150 bars.

4. Procédé selon l'une des revendications 1 à 3, caractérisé en ce qu'on hydrogène à des températures comprises entre 80 et 100°C.

5. Procédé selon l'une des revendications 1 à 4, caractérisé en ce qu'on hydrogène après addition d'un alcool, de préférence le méthanol.